# EUROPEAN PATENT APPLICATION

(11) **EP 2 716 769 A2**
(43) Date of publication of application: **09.04.2014**
(21) Application number: 13183209.9
(22) Date of filing: 15.05.2009
(51) Int. Cl.: C12Q 1/68, G01N 33/92, A61K 31/00, A61P 25/18

(54) **Method of screening antipsychotic drugs**

(30) Priority: 16.05.2008 US 54086 P
(62) Divisional of application: 09747746.7
(71) Applicant: Vanda Pharmaceuticals Inc., Rockville NY 20850 (US)
(72) Inventor: Lavedan, Christian, MD, MD Maryland 20854 (US); Licamele Louis, MD, MD Maryland 20878 (US); Polymeropoulos, Mihael H, MD, MD Maryland 20854 (US)
(74) Representative: Atkinson, Jennifer

(57) **Abstract**

The invention concerns a method of screening for compounds having antipsychotic activity that comprises identifying a molecular signature for compounds known to have antipsychotic activity and then screening compounds not known to have the antipsychotic activity to identify compounds that have the molecular signature and therefore have the given antipsychotic activity. This method can also be used to monitor patients undergoing treatment.

## Description

### CROSS-REFERENCE TO RELATED APPLICATIONS

This application claims the benefit of co-pending US Provisional Patent Application No. 61/054,086, filed 16 May 2008, which is hereby incorporated herein.

### BACKGROUND

The underlying molecular etiology of schizophrenia remains poorly understood although the dopamine hypothesis has been the most influential for decades. In the dopamine hypothesis, alteration of the homeostasis of neurotransmitters including dopamine and serotonin is believed to result in the production of the symptoms of the disease (Toda and Abi-Dargham 2007). Other mechanisms of the pathophysiology of schizophrenia have been proposed including a neurodevelopmental hypothesis (Nasrallah 1993) where alterations of membrane phospholipids could play a major part (Horrobin 1998), a role for glutamate (Goff and Coyle 2001) and the muscarinic cholinergic system (Raedler et al. 2007), and an inflammation of the microvasculature system (Hanson and Gottesman 2005). There is also much evidence that schizophrenia is highly heritable and may be caused by several interacting susceptibility genetic loci and environmental factors (Van Os and Sham 2003;Freedman et al. 2001;Tsuang et al. 2004) However, despite intense research efforts to identify genetic defects in the neurotransmitter systems in patients and families with schizophrenia, no consistent genetic alteration has been identified to date.

A number of pharmacological agents are available today in the class of antipsychotics which are used for management of the disease symptoms. They share the ability to block the effect of neurotransmitters through the binding of an array of receptors. The antipsychotics

### SUMMARY

In brief, the invention is a method of screening for compounds having a given activity that comprises identifying a molecular signature for compounds known to have the given activity and then screening compounds not known to have the given activity to identify compounds that have that molecular signature and therefore have the given activity. In illustrative embodiments, this methodology is applied to define molecular signatures for antipsychotics and SERMs and can be used, for example, to screen compounds for such activities, to monitor patients undergoing treatment, and to treat patients with compounds not previously known to have such activities.

The invention may be further described by the following numbered paragraphs:
1. A method of preventing or treating a disorder that is amenable to treatment with an antipsychotic, or with a SERM, that comprises internally administering to a patient an agent, other than a known typical or atypical antipsychotic, or a known SERM, respectively, that alters lipid homeostasis.
2. The method of paragraph 1 wherein the agent modulates the ratio of polyunsaturated to saturated fatty acids and/or cholesterol content and that thereby alter the fluidity of cell membranes of neurons and supporting cells, resulting in changes in neuronal connectivity.
3. A method of preventing or treating a disorder that is amenable to treatment with an antipsychotic that comprises internally administering to a patient an agent, other than a known typical or atypical antipsychotic, wherein the agent upregulates one or more of the genes listed in Table 2.
4. A method of preventing or treating a disorder that is amenable to treatment with an antipsychotic that comprises internally administering to a patient an agent, other than a known typical or atypical antipsychotic, wherein the agent upregulates one or more of the following genes: INSIG1, SCD, FADS2, LDLR, FADS1, FDPS, ACAT2, FDFT1, CYP51A1, FASN, DHCR7, RAB26, TM7SF2, SATB1, FAM117A, GPNMB, NUPR1, VAC14, and LSS.
5. The method of paragraph 4 wherein the agent upregulates one or more of the following genes: INSIG1, SCD, FADS2, LDLR, FADS1, FDPS, ACAT2, FDFT1, CYP51A1, FASN, DHCR7, RAB26, and TM7SF2.
6. The method of paragraph 4 wherein the agent upregulates one or more of the following genes: INSIG1, SCD, LDLR, FADS1, and CYP51A1.
7. The method of paragraph 4 wherein the agent upregulates one or more of the following genes: LDLR, INSIG1, FADS1, SCD, LSS, CYP51A1, VAC14, NUPR1, GPNMB, FAM117A, SATB1 and DDR2.
8. The method of paragraph 4 wherein the agent upregulates one or more of the following genes: LDLR, INSIG1, FADS1, SCD, LSS, CYP51A1, and GPNMB.
9. The method of paragraph 3 in which upregulation is defined as an amplitude of 0.4 or higher.
10. The method of paragraph 3 in which the agent has a KS score of .99 or higher, or of .995 or higher, or of .997 or higher, or of .998 or higher, or of .999 or higher for the probes listed in Table 1.
11. The method of paragraph 3 wherein two or more genes are ranked by change in expression and the ranking of said two or more genes is as shown in Table 1, Table 2, or Table 3.
12. A method for monitoring a patient undergoing treatment with an antipsychotic or a SERM that comprises monitoring for upregulation of a gene that alters lipid homeostasis.
13. The method of paragraph 12 that comprises monitoring the ratio of polyunsaturated to saturated fatty acids and/or cholesterol content and thereby the fluidity of cell membranes of neurons and supporting cells, resulting in changes in neuronal connectivity.
14. A method for monitoring a patient undergoing treatment with an antipsychotic that comprises monitoring for upregulation of one or more of the genes listed in Table 2.
15. A method for monitoring a patient undergoing treatment with an antipsychotic that comprises monitoring for upregulation of one or more of the following genes: INSIG1, SCD, FADS2, LDLR, FADS1, FDPS, ACAT2, FDFT1, CYP51A1, FASN, DHCR7, RAB26, TM7SF2, SATB1, FAM117A, GPNMB, NUPR1, VAC14, and LSS.
16. The method of paragraph 15 that comprises monitoring for upregulation of one or more of the following genes: INSIG1, SCD, FADS2, LDLR, FADS1, FDPS, ACAT2, FDFT1, CYP51A1, FASN, DHCR7, RAB26, and TM7SF2.
17. The method of paragraph 15 that comprises monitoring for upregulation of one or more of the following genes: INSIG1, SCD, LDLR, FADS1, and CYP51A1.
18. The method of paragraph 15 that comprises monitoring for upregulation of one or more of the following genes: LDLR, INSIG1, FADS1, SCD, LSS, CYP51A1, VAC14, NUPR1, GPNMB, FAM1 17A, SATB1 and DDR2.
19. The method of paragraph 15 that comprises monitoring for upregulation of one or more of the following genes: LDLR, INSIG1, FADS1, SCD, LSS, CYP51A1, and GPNMB.
20. The method of paragraph 14 in which upregulation is defined as an amplitude of about 0.4 or higher.
21. The method of paragraph 14 in which the agent has a KS score of .99 or higher, or of .995 or higher, or of .997 or higher, or of .998 or higher, or of .999 or higher for the probes listed in Table 1.
22. The method of paragraph 14 wherein two or more genes are ranked by change in expression and the ranking of said two or more genes is as shown in Table 1, Table 2, or Table 3.
23. A method of screening agents for antipsychotic or SERM activity that comprises screening agents for their ability to upregulate a gene that alters lipid homeostasis.
24. The method of paragraph 21 that comprises screening agents for their ability to modulate the ratio of polyunsaturated to saturated fatty acids and/or cholesterol content and thereby to alter the fluidity of cell membranes of neurons and supporting cells, resulting in changes in neuronal connectivity.
25. A method of screening agents for antipsychotic activity that comprises screening agents for their ability to upregulate one or more of the genes listed in Table 2.
26. A method of screening agents for antipsychotic activity that comprises screening agents for their ability to upregulate one or more of the following genes: INSIG1, SCD, FADS2, LDLR, FADS1, FDPS, ACAT2, FDFT1, CYP51A1, FASN, DHCR7, RAB26, TM7SF2, SATB1, FAM117A, GPNMB, NUPR1, VAC14, and LSS.
27. The method of paragraph 26 that comprises screening agents for their ability to upregulate one or more of the following genes: INSIG1, SCD, FADS2, LDLR, FADS1, FDPS, ACAT2, FDFT1, CYP51A1, FASN, DHCR7, RAB26, and TM7SF2.
28. The method of paragraph 26 that comprises screening agents for their ability to upregulate one or more of the following genes: INSIG1, SCD, LDLR, FADS1, and CYP51A1.
29. The method of paragraph 26 that comprises screening agents for their ability to upregulate one or more of the following genes: LDLR, INSIG1, FADS1, SCD, LSS, CYP51A1, VAC14, NUPR1, GPNMB, FAM117A, SATB1 and DDR2.
30. The method of paragraph 26 that comprises screening agents for their ability to upregulate one or more of the following genes: LDLR, INSIG1, FADS1, SCD, LSS, CYP51A1, and GPNMB.
31. The method of paragraph 25 in which upregulation is defined as an amplitude of about 0.4 or higher.
32. The method of paragraph 25 in which the agent has a KS score of .99 or higher, or of .995 or higher, or of .997 or higher, or of .998 or higher, or of .999 or higher for the probes listed in Table 1.
33. The method of paragraph 25 wherein two or more genes are ranked by change in expression and the ranking of said two or more genes is as shown in Table 1, Table 2, or Table 3.
34. A method of preventing or treating a disorder that is amenable to treatment with an antipsychotic that comprises internally administering to a patient an agent, other than a known typical or atypical antipsychotic, wherein the agent upregulates one or more of the genes listed in Table 1, Table 2, Table 3 or in both Tables 1 and 2.
35. The method of paragraph 34 in which the agent has a KS score of .99 or higher, or of .995 or higher, or of .997 or higher for the probes listed in Table 1.
36. The method of paragraph 34 wherein two or more genes are ranked by change in expression and the ranking of said two or more genes is as shown in Table 1, Table 2, or Table 3.
37. A method of preventing or treating a disorder that is amenable to treatment with a SERM that comprises internally administering to a patient an agent, other than a known SERM, wherein the agent upregulates one or more of the genes listed in Table 1, Table 2, Table 3 or in both Tables 1 and 2.
38. The method of paragraph 37 in which the agent has a KS score of .99 or higher, or of .995 or higher, or of .997 or higher for the probes listed in Table 1.
39. The method of paragraph 37 wherein two or more genes are ranked by change in expression and the ranking of said two or more genes is as shown in Table 1, Table 2, or Table 3.
40. A method of screening agents for SERM activity that comprises screening agents for their ability to upregulate one or more of the genes listed in Table 1, Table 2, Table 3 or in both Tables 1 and 2.
41. The method of paragraph 40 in which the agent has a KS score of .99 or higher, or of .995 or higher, or of .997 or higher for the probes listed in Table 1.
42. The method of paragraph 40 wherein two or more genes are ranked by change in expression and the ranking of said two or more genes is as shown in Table 1, Table 2, or Table 3.
43. A method for monitoring a patient undergoing treatment with an antipsychotic that comprises monitoring for upregulation of one or more of the genes listed in Table 1, Table 2, Table 3 or in both Tables 1 and 2.
44. The method of paragraph 43 in which the agent has a KS score of .99 or higher, or of .995 or higher, or of .997 or higher for the probes listed in Table 1.
45. The method of paragraph 43 wherein two or more genes are ranked by change in expression and the ranking of said two or more genes is as shown in Table 1, Table 2, or Table 3.

### BRIEF DESCRIPTION OF THE FIGURES

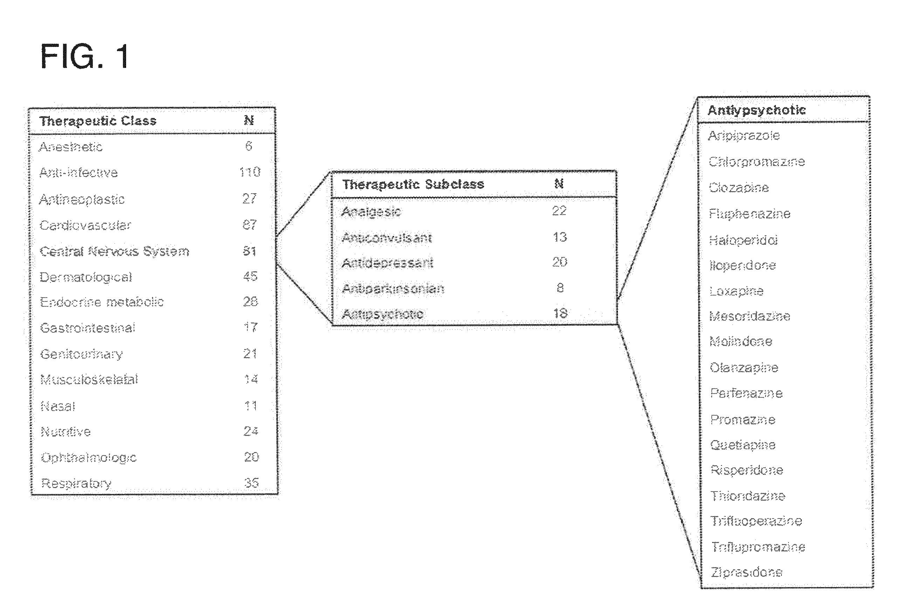

Figure 1. Overview of compounds profiled across therapeutic classes and sub-classes. Therapeutic classes and subclasses are listed as defined by the The Physicians' Desk Reference (PDR, Thomson Healthcare at Montvale, NJ, USA), with the number of drugs profiled (N). Some drugs are listed in more than one therapeutic class or subclass.

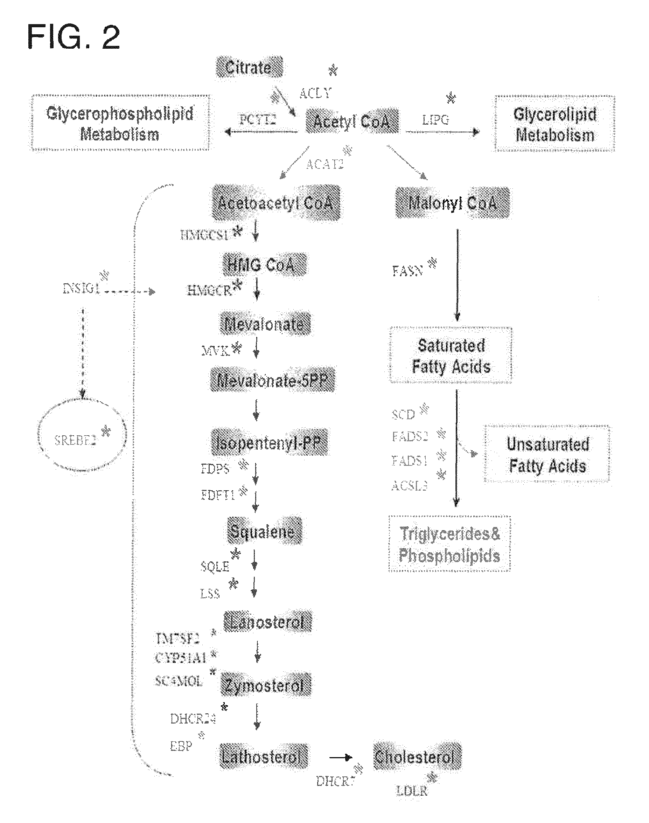

Figure 2. Effect of Antipsychotics on Fatty Acid and Cholesterol Biosynthesis. Pathways of Fatty Acid and Cholesterol Biosynthesis are shown with the major metabolite intermediates and enzymatic reactions (arrows). Genes coding for relevant enzymes, regulators, or transporters are shown with a colored asterisk: red or blue when the corresponding probe set(s) was (were) in the top 20 or top 100, respectively, of the antipsychotic profile. Note that it is a simplified representation of these complex pathways; not all reactions, enzymes, genes, or intermediates are represented.

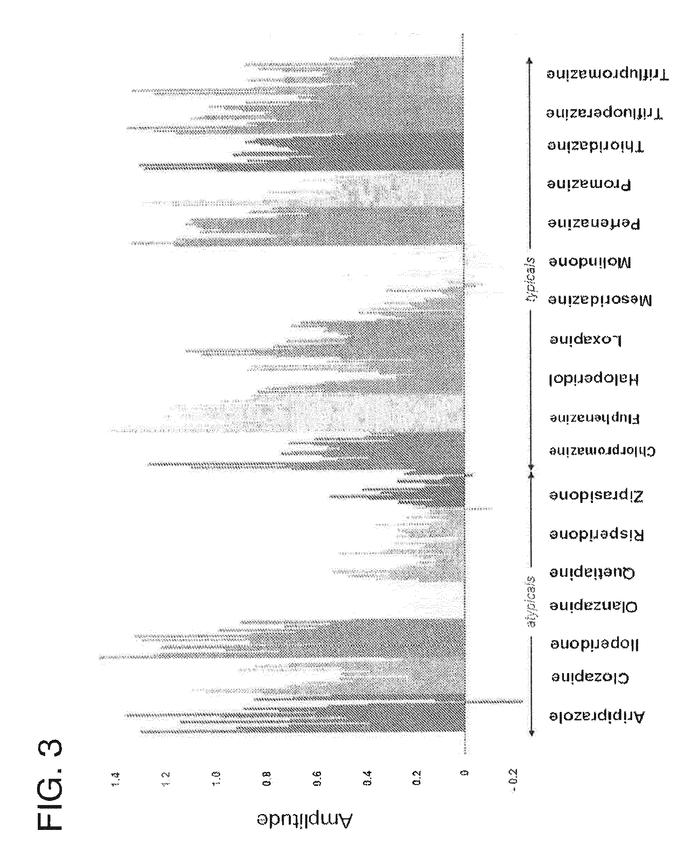

Figure 3. Expression changes of top ranked probe sets in the antipsychotic group profile. Change in expression between drug treatment and vehicle control is shown by the mean amplitude (Y axis) for each the top 20 ranked probe sets (left to right on the X axis, ordered by rank) for each antipsychotic tested. The mean amplitude graphed is the average amplitude across replicates.

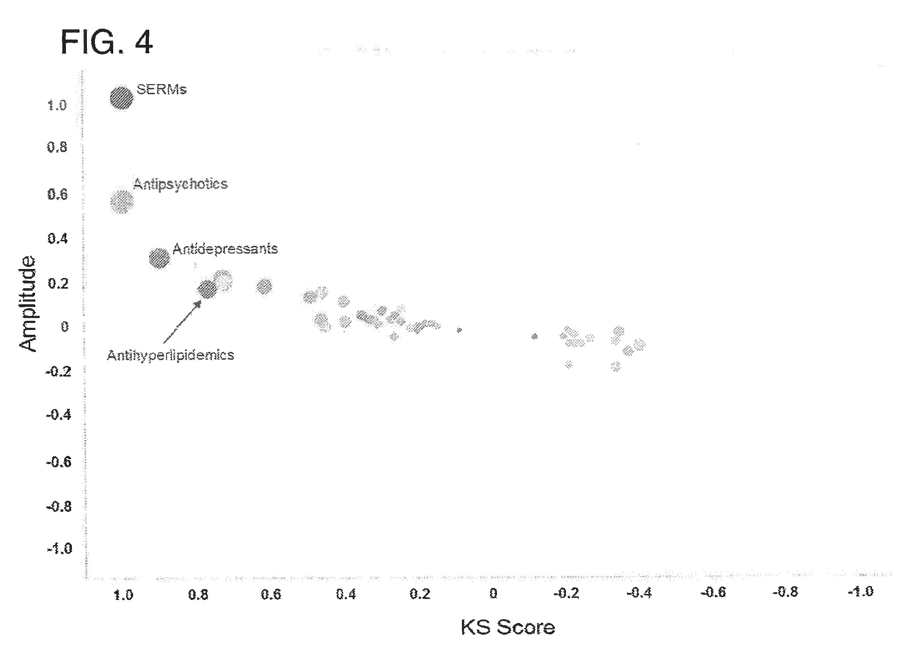

Figure 4. Visualization of the antipsychotic gene expression signature across a library of compounds. The average amplitude of the 20 probe sets from the antipsychotic signature is represented by a dot for each group of drugs. The size of each dot corresponds to the absolute similarity score (KS value) of these 20 probe sets. Only groups of ≥5 drugs from a therapeutic class or subclass as defined by the PDR are represented here, with the addition of the SERMs which are listed by the PDR as either antineoplastic or endocrine-metabolic agents. Actual values are given in Table 6.

### DETAILED DESCRIPTION

For decades, the dopamine hypothesis has gained the most attention in an attempt to explain the origin and the symptoms of schizophrenia. While this hypothesis offers an explanation for the relationship between psychotic symptoms and dopamine kinetics, it does not provide a direct explanation of the etiology of schizophrenia which remains poorly understood. Consequently, current antipsychotics that target neurotransmitter receptors, have limited and inconsistent efficacy. To gain insights into the mechanism of action of these drugs, we studied the expression profile of 12,490 human genes in a cell line treated with 18 antipsychotics, and compared it to that of a library of 448 other compounds used in a variety of disorders. Analysis reveals a common effect of antipsychotics on the biosynthesis and regulation of fatty acids and cholesterol, which is discussed in the context of a lipid hypothesis where alterations in lipid homeostasis might underlie the pathogenesis of schizophrenia. This finding may help research aimed at the development of novel treatments for this devastating disease.

In an effort to discover molecular signatures of pharmaceutical agents, including antipsychotics, we have screened 466 compounds that belong to 14 different therapeutic classes (Figure 1), in a human retinal pigment epithelia cell line (ARPE-19) and studied the resulting gene expression changes across 12,490 genes. The choice of the ARPE-19 cell line is particularly well suited for the study of compounds that affect neuronal type cells, in particular antipsychotics. It expresses a variety of cell surface receptors that include the dopamine receptor D2, the serotonin receptors 1A, 2A, and 2C, the muscarinic receptor M3, and the histamine receptor H1 (Dr. Maria A. DeBernardi, personal communication). Furthermore several antipsychotics have been associated with degenerative retinopathies (Fornaro et al. 2002).

We describe here the discovery of an "antipsychotic signature" which gives insights into the therapeutic effect of these drugs.

### Experimental/Materials and methods.

### Cell culture and drug treatment.

The retinal pigment epithelia cell line, ARPE-19/HPV-16, was chosen to establish a database of drug profiles because it is from non-cancerous human origin, with a normal karyotype, and can easily be grown as monolayer in 96-well plates. H4 is a hypertriploid cell line from glioblastoma origin, which was used only for independent replication. Cell lines were propagated according to supplier's specifications (ATCC Manassas, VA). Compounds were obtained from Sigma (St. Louis, MO) or Vanda Pharmaceuticals (Rockville, MD). Cells were aliquoted to 96-well plates (∼2x10e5 cells/well) and incubated for 24 hrs prior to providing fresh media with a drug, or the drug vehicle (water, dimethyl sulfoxide, ethanol, methanol, or phosphate-buffered saline solution). Drugs were diluted 1000 fold in buffered Advanced D-MEM/F-12 culture medium (Invitrogen, Carlsbad, CA) containing non-essential amino acids and 110 mg/L sodium pyruvate. In these conditions, no significant changes of pH were expected, which was confirmed by the monitoring of the pH indicator present in the medium. A final 10µM drug concentration was chosen because it is believed to fit in the range of physiological relevance, and has been used in other cell line studies (Ferno et al. 2006;Lamb et al. 2006). Microscopic inspection of each well was conducted at the end of the treatment to discard any instance where cells had morphological changes consistent with apoptosis, and to verify that the drug had not precipitated in the culture medium.

### Gene expression profiles.

Cells were harvested 24 hrs after treatment and RNA extracted using the RNeasy 96 protocol (Qiagen, Valencia, CA). Gene expression for 22,238 probe sets of 12,490 genes was generated with U133A2.0 microarrays following the manufacture's instructions (Affymetrix, Santa Clara, CA). Antipsychotics were profiled in duplicate or triplicate, with multiple vehicle controls on each plate. A total of 708 microarrays were analysed including 74 for the 18 antipsychotics, 499 for the other 448 compounds, and 135 for vehicle controls.

Each drug treatment-vehicle control pair (treatment instance) was represented by a non-parametric rank-ordered list constructed as follows, similarly to the method described by Lamb and colleagues (Lamb et al. 2006). The raw scan data were first converted to average difference values using MAS 5.0 (Affymetrix). The average difference values of both treatment and control data were set to a minimum threshold of 50 if below 50. For each treatment instance, all probe sets were then ranked based on their amplitude, or level of expression relative to the vehicle control (or average of controls when more than one was used). Amplitude was defined as the ratio of expression (t-v)/[(t+v)/2] where t corresponds to treatment instance and v to vehicle instance.

### Data Analysis.

Each drug group profile was created using our novel Weighted Influence Model, Rank of Ranks (WIMRR) method which underscores the rank of each probe set across the entire gene expression profile rather than the specific change in expression level. WIMRR takes the average rank of each probe set across all of the members of the group and then re-ranks the probe sets from smallest average rank to largest average rank.

A gene-set enrichment metric based on the Kolmogorov-Smirnov (KS) statistic was used in a similar fashion by Lamb and colleagues (Lamb et al. 2006). Specifically, for a given set of probes, the KS score gives a measure of how up (positive) or down (negative) the set of probes occurs within the profile of another treatment instance.

### Results

We analysed the gene expression group profile of 18 drugs used to treat patients with schizophrenia, which included 11 typical and 7 atypical antipsychotics (Figure 1). In order to detect a common molecular signature shared by these compounds, we determined with the WIMRR method the probe sets that were the most consistently up-regulated (highest ranks) or down-regulated (lowest ranks) by the group of these 18 antipsychotic agents, and looked for possible involvement in similar metabolic pathways using literature and other publicly available resources.

### Over-expression of genes implicated in lipid homeostasis

Nineteen of the first 20 (95%) ranked probe sets in the antipsychotic group profile correspond to 13 genes involved in fatty acids and cholesterol biosynthesis, or in phospholipid metabolism (Table 1 and Figure 2). The insulin induced gene 1 *(INSIGI)* gene encodes an endoplasmic reticulum membrane protein that is a main regulator of cholesterol concentrations in cells. The stearoyl-CoA desaturase *(SCD)* gene codes for the rate-limiting enzyme that catalyzes the conversion of saturated long-chain fatty acids into monounsaturated fats that are the major components of triglycerides, phospholipids, and cholesterol esters. The fatty acid desaturases 1 and 2 *(FADS1*, *FADS2)* and the famesyl diphosphate synthase *(FASN)* arc central to the biosynthesis of fatty acids, and the acetyl-Coenzyme A acetyltransferase 2 *(ACAT2)* plays a major role in lipid metabolism. The genes for low density lipoprotein receptor *(LDLR),* farnesyl-diphosphate farnesyltransferase 1 *(FDFTI),* cytochrome P450, family 51, subfamily A, polypeptide 1 *(CYP51A1), 7-*dehydrocholesterol reductase *(DHCR7),* transmembrane 7 superfamily member 2 *(TM7SF2),* and emopamil binding protein, sterol isomerase *(EBP)* all code for key proteins of the steroid biosynthesis. The phosphate cytidylyltransferase 2, ethanolamine gene *(PCYT2)* codes for an enzyme of the biosynthetic pathway of phosphatidylethanolamine, a major membrane phospholipid. One probe set recognized the gene encoding RAB26, member RAS oncogene family *(RAB26),* that belongs to a family of proteins considered key regulators of intracellular vesicle transport during exocytosis; most Rab proteins bind lipids, which is essential for membrane attachment.

This lipid homeostasis signature activated by the group of antipsychotics appears even stronger upon a comprehensive analysis beyond the top 20 probe sets, which revealed the up-regulation of more genes from the same biological pathways (Figure 2 and Table 2). We also observed that typical and atypical antipsychotics had a similar effect on lipid homeostasis (Figure 3). The stronger up-regulation of these 20 probe sets was observed with fluphenazine, perphenazine and iloperidone. Molindone and mesoridazine had the lowest effect. Differences between drugs may reflect their unique physicochemical properties, receptor binding affinities, or distinct potency as previously noted (Ferno et al. 2006).

We confirmed the effect on lipid homeostasis by profiling the same 18 antipsychotics in a different human cell line from glioblastoma origin (H4). The top 20 ranked probe sets of the antipsychotic profile obtained in the H4 cell line include probe sets for the *LDLR, INSIG1, FADS1, SCD, CYP51A1,* as well as the gene encoding the lanosterol synthase *(LSS)* (Table 3). This result indicates that the effect of antipsychotics on lipid homeostasis is not limited to a particular cell culture system.

Importantly, our study demonstrates for the first time that antipsychotics not only activate genes involved in lipid homeostasis but do this preferentially from all other genes. An analysis of gene ontology (GO) biological process terms (Lomax 2005) with the L2L tool (Newman and Weiner 2005) showed that our observation was statistically significant and not biased by the number of probe sets linked to this biological process (Table 3). In the group profile of antipsychotics, "lipid biosynthetic process" was the GO term most significantly associated with the top 20 and top 100 probes sets (p=5.23E-16 and 6.20E-33, respectively, after Bonferroni correction for the analysis of 2,075 GO terms). In contrast, the genes most down-regulated by the group of antipsychotics included genes from various biological processes, with no clear common significance (Table 4). The GO term most significantly associated with the bottom 20 probes sets was "mitosis" (p=1.06E-03 after Bonferroni correction).

### Common signature shared by antipsychotics and estrogen receptor modulators

By comparing gene expression group profiles of all drug classes, it was further discovered that the group of selective estrogen receptor modulators (SERMs),which included tamoxifen, raloxifene, and clomiphene, affected lipid homeostasis in a manner similar to antipsychotics (KS scores of 0.997 and 0.806 for the top 20 and 100 probe sets, respectively) (Figure 4 and Table 5). In addition, the antipsychotic signature of the top 20 or top 100 probe sets appears unique among all other therapeutic classes and sub-classes of more than 400 compounds studied, with only a partial overlap with antidepressants (KS scores of 0. 898 and 0.738, respectively) and antihyperlipidemic agents (KS scores of 0.768 and 0.522, respectively) (Figure 4).

### Discussion

### Supporting evidence for an effect of antipsychotics on lipid homeostasis

Our discovery of a common gene expression signature of antipsychotics affecting fatty acids and cholesterol biosynthesis is in agreement with a number of independent observations, which substantiate that the drug concentration used in our in-vitro experiment (10uM) is physiologically relevant.

A genome-wide screen of *Saccharomyces cerevisiae* heterozygotes had previously revealed that the antipsychotics haloperidol, chlorpromazine, and trifluoperazine had a strong effect on genes involved in yeast fatty acid biosynthesis *(OLE1,* the ortholog of the human *SCD),* sterol biosynthesis *(SET6* and *ERG3* the ortholog of the human the sterol-C5-desaturase gene *SC5DL),* or phospholipid transport *(NEO1* highly homologous to human phospholipid transporting ATPases) (Lum et al. 2004).

To date, no animal study designed to identify gene expression changes common to a large number of antipsychotics has been reported; however, several groups have reported genome-wide expression profiling of a limited number of antipsychotics. Three studies in the rat prefrontal cortex after chronic administration of risperidone (Chen and Clien 2005) olanzapine (Fatemi et al. 2006) or aripiprazole (Cheng et al. 2008) showed no consistent gene expression changes across the different antipsychotics. A recent analysis of the effect of 3 antipsychotics (haloperidol, clozapine and olanzapine) on changes in the mouse brain showed a limited level of similarity between the 3 drugs (Duncan et al. 2008). These studies did not report changes in genes involved in lipid homeostasis. In another study, one of the most notable effect of clozapine and haloperidol in the mouse striatum was reported to be the differential expression of genes involved in lipid metabolism (Thomas et al. 2003). It is unclear at this time if these apparent inconsistencies are due to different experimental designs and conditions (model system, length of drug exposure...) or reflect changes specific to individual drugs or group of drugs. However, effect of antipsychotics on lipids has been well documented in schizophrenia patients.

In particular, further support for our findings comes from the work of other researchers that aimed at understanding the molecular origin of the known metabolic side effect of antipsychotics that includes increased weight gain and propensity to adiposity and insulin resistance (Newcomer 2007). In particular, Ferno et al. have analysed the activation of 9 genes by several antipsychotics, namely clozapine, olanzapine, haloperidol, chlorpromazine, risperidone and ziprasidone, in a human glioma cell line. All 9 genes were among the top genes up-regulated in our antipsychotic signature (Table 2); they included *SCD, FASN, DHCR7, LDLR, FDPS, ACAT2, SC5DL,* the 3-hydroxy-3-methylglutaryl-Coenzyme A reductase *(HMGCR),* and the 3-hydroxy-3-methylglutaryl-Coenzyme A synthase 1 *(HMGCS1).* Up-regulation *of SCD* and *FASN* has also been demonstrated in blood cells from patients with psychotic disorders treated with olanzapine (Vik-Mo et al. 2008). An extensive analysis of lipid metabolites has shown that medication-free schizophrenic patients had significantly lower levels of plasma phosphoethanolamines (PE) than control subjects (Kaddurah-Daouk et at. 2007). In this study, it was also determined that 2-3 weeks of antipsychotic treatment led to significant increases in plasma levels of certain lipid classes, namely PE, phosphatidylcholine (PC), and triacylglycerols (TG) with olanzapine, PE, PC, and lysophosphatidylcholine (LY) with risperidone; aripiprazole had only a minimal effect on PE concentrations. Based on specific changes observed in the lipid biosynthetic pathway, the authors hypothesized a possible increase in Δ6 desaturase and decrease in Δ5 desaturase following treatment with olanzapine or risperidone. In the cell line experiment presented here, an over-expression of RNAs for both the Δ5 and Δ6 desaturase genes (FADS1 and FADS2) was observed with most antipsychotics, including olanzapine and risperidone (Table 1, Figure 3)"

### Therapeutic effect of antipsychotics

We propose that the activation by antipsychotics of genes associated with lipid homeostasis is not just a common off target effect of these drugs but rather the common central mechanism by which they achieve their antipsychotic activity. It is interesting to note that several antipsychotics which share the gene signature do not have the same type of metabolic or lipid-related adverse event profile; some antipsychotics are known to have a significant increased risk for weight gain and hyperlipidemia, (i.e. clozapine, olanzapine) while others pose a much lower risk for these side effects (i.e. risperidone, ziprasidone). Nevertheless, these 4 drugs share a common gene expression signature related to lipid homeostasis. This signature is also shared by SERMs which do not have the same type of metabolic-related adverse events as antipsychotics. For example, it has been shown that concentrations of cholesterol can be significantly reduced in postmenopausal women treated with raloxifene (Dayspring T et al., Metabolism 2006).

It is plausible that, in vivo, antipsychotics, ultimately achieve their therapeutic effect by altering lipid homeostasis. It has been proposed that the ratio of polyunsaturated to saturated fats in the membrane is associated with metabolic rate (Hulbert 2007). We suggest that modulation of the ratio of polyunsaturated to saturated fatty acids and cholesterol content by antipsychotic drugs alters the fluidity of cell membranes of neurons and supporting cells, resulting in changes in neuronal connectivity. The up-regulation of *SCD, FADS1* and *FADS2* points to a central role of the regulation of the composition of mono- and poly-unsaturated fats which are key components of cellular membranes and directly involved with membrane fluidity. Re-constitution of the lipid membrane composition to achieve improvement of schizophrenia symptoms could explain the time lag of the therapeutic effect seen with these agents (Emsley et al. 2006).

Our observation of a similar effect of antipsychotics and SERMs on genes involved in fatty acids metabolism and cholesterol biosynthesis points to a potential implication of the estrogen pathway in the antipsychotic efficacy response. It has been previously reported that dopamine can activate estrogen receptors (Power et al. 1991), which could explain why antipsychotics and SERMs exhibit a similar molecular effect in our experiment. Modulation of the estrogen receptor has been shown to result in the activation of SREBP responsive genes in the cholesterol biosynthetic pathway (Wang et al. 2006;Ntambi 1999) such as *SCD, FDPS, DHCR7, LDLR, ACAT2* and *FASN,* all up-regulated by antipsychotics in our experiment. Interestingly, it has been shown in rats that tamoxifen can prevent obesity induced by the antipsychotic sulpiride (Baptista et al. 1997). Moreover, several clinical studies have shown that tamoxifen can reduce mania symptoms in patients with bipolar disorder (Kulkarni et al. 2006;Zarate, Jr. et al. 2007;Yildiz et al. 2008). Symptoms of psychosis and cognitive functioning were also shown to improve in women affected with schizophrenia who were treated with oestradiol or raloxifene (Kulkarni et al. 2008).

### A lipid hypothesis of schizophrenia

Several clinical observations have provided convergent evidence for a lipid hypothesis of schizophrenia. We propose that an aberration of lipid homeostasis may represent a key deficit in the etiology of this disease and possibly other behavioral disorders. It is relevant to note that the onset of schizophrenia usually occurs in late adolescence or early adulthood (Harrop and Trower 2001), at a period of development when significant changes in fatty acid composition are taking place in the cerebral cortex (Carver et al. 2001). Indeed, it has been shown that drug-naïve patients with schizophrenia have increased intra-abdominal fat, impaired fasting glucose tolerance and are more insulin resistant than healthy individuals (Thakore et al. 2002;Ryan et al. 2003). Several studies have demonstrated a decrease of phospholipids and polyunsaturated fatty acids in postmortem brain as well as in peripheral membranes in schizophrenia (Yao et al. 2000;Horrobin et al. 1991;Komoroski et al. 2001;Schmitt et al. 2004). Abnormalities in phospholipase A2 activity which plays an essential role in the breakdown of phospholipids, and in the metabolism of prostaglandins that are derived enzymatically from fatty acids, have also been reported (Smcsny et al. 2005;Maida et al. 2006;Gattaz et al. 1995). Of particular interest is the observation of a reduction of essential polyunsaturated fatty acids in the red blood cell membrane of patients with first episode schizophrenia who had not yet been treated with antipsychotics (Reddy et al. 2004), which indicates that these deficits in fatty acids precede drug treatment.

Our findings suggest also that the estrogen pathway is involved in the therapeutic effect of antipsychotics, which is in agreement with the notion that estrogen levels play a role in the etiology and/or the severity of symptoms. Recently, variants in the estrogen receptor alpha gene which may contribute to risk for schizophrenia have been identified (Weickert et al. 2008). A possible protective effect of estrogen has been proposed based on the observation that, relative to men, women show a delay in onset age of schizophrenia, with a second onset peak after age 44 years (Grigoriadis and Seeman 2002). It has also been reported that schizophrenia symptoms in women frequently vary with the menstrual cycle, worsening during phases of low estrogen (Grigoriadis and Seeman 2002).

Another observation consistent with the involvement of fatty acid metabolism in schizophrenia comes from a recent clinical study examining the efficacy and safety of the novel antipsychotic iloperidone, in which we have shown that patients carrying a null mutation in the ciliary neurotrophic factor *(CNTF)* gene have a high placebo response, indicating an inherent ability of overcoming an acute exacerbation of schizophrenia (Lavedan et al. 2008). A CNTF peptidomimetic (axokine) was shown to significantly decrease the expression of the *SCD* gene in adipocytes, a function shared by leptin (Sleeman et al. 2003). Interestingly, we showed that *SCD* was one of most consistently up-regulated genes by the group of antipsychotics. Therefore, it is conceivable that patients with schizophrenia who carry the *CNTF* null mutation have a higher constitutive expression of the *SCD* gene, which could explain their ability to better overcome an acute exacerbation, at least in the short term, than patients with the normal CNTF protein.

In this proposed lipid hypothesis, we would expect that the administration of certain fatty acids could improve schizophrenia symptoms. Support towards this aspect of the lipid hypothesis comes from reports of nutritional deficiencies including omega-3 fatty acids in mental disorder patients (Hibbeln 1998;Rudin 1982;Rudin 1981), low incidence rate and better prognoses due to consumption of omega-3 fatty acids (Hibbeln 1998;Reis and Hibbeln 2006;Christensen and Christensen 1988), and the positive therapeutic effects of administration of polyunsaturated omega-3 fatty acids in patients with schizophrenia (Peet 2003;Puri et al. 2000;Richardson et al. 1999;Emsley et al. 2002;Yao et al. 2004;Sivrioglu et al. 2007;Richardson AJ. 2003;Richardson AJ. 2003). Moreover, a study by McNamara et al. identified deficits of omega-3 fatty acids in the orbitofrontal region of brains of patients with schizophrenia; these deficits were partially restored in patients treated with antipsychotics (McNamara et al. 2007).

In conclusion, we suggest that an array of alterations in genes responsible for lipid homeostasis may be involved in the pathogenesis of the disease. We understand that further research in the lipid hypothesis of schizophrenia may provide fundamental knowledge of the gene-environment interactions in the causation and course of the disease. Finally it is our hope that the finding of a common effect of current antipsychotics on genes involved in lipid will spark research aimed at the development of new treatments for this devastating disease.

**Table 1. Top 20 ranked probe sets of the antipsychotic group profile in the ARPE-19 cell line**

| Rank | Probe set | Gene | Description |
|---|---|---|---|
| 1 | 201627_s_at | *INSIG1* | insulin induced gene 1 |
| 2 | 200831_s_at | *SCD* | stearoyl-CoA desaturase (delta-9-desaturase) |
| 3 | 200832_s_at | *SCD* | stearoyl-CoA desaturase (delta-9-desaturase) |
| 4 | 211162_x_at | *SCD* | stearoyl-CoA desaturase (delta-9-desaturase) |
| 5 | 202218_s_at | *FADS2* | fatty acid desaturase 2 |
| 6 | 202067_s_at | *LDLR* | low density lipoprotein receptor |
| 7 | 208964_s_at | *FADS1* | fatty acid desaturase 1 |
| 8 | 201275_at | *FDPS* | farnesyl diphosphate synthase |
| 9 | 201625_s_at | *INSIG1* | insulin induced gene 1 |
| 10 | 202068_s_at | *LDLR* | low density lipoprotein receptor |
| 11 | 209608_s_at | *ACAT2* | acetyl-Coenzyme A acetyltransferase 2 |
| 12 | 210950_s_at | *FDFT1* | farnesyl-diphosphate farnesyltransferase 1 |
| 13 | 216607_s_at | *CYP51A1* | cytochrome P450, family 51, subfamily A, polypeptide 1 |
| 14 | 212218_s_at | *FASN* | fatty acid synthase |
| 15 | 201790_s_at | *DHCR7* | 7-dehydrocholesterol reductase |
| 16 | 219562_at | *RAB26* | RAB26, member RAS oncogene family |
| 17 | 210130_s_at | *TM7SF2* | transmembrane 7 superfamily member 2 |
| 18 | 208962_s_at | *FADS1* | fatty acid desaturase 1 |
| 19 | 209577_at | *PCYT2* | phosphate cytidylyltransferase 2, ethanolamine |
| 20 | 202735_at | *EBP* | emopamil binding protein (sterol isomerase) |

**Table 2. Top 100 ranked probe sets of the antipsychotic group profile in the ARPE-19 cell line**

| Rank | Probe set | Gene | Description |
|---|---|---|---|
| 1 | 201627_s_at | *INSIG1* | insulin induced gene 1 |
| 2 | 200831_s_at | *SCD* | stearoyl-CoAdesaturase (delta-9-desaturase) |
| 3 | 200832_s_at | *SCD* | stearovl-CoA desaturase (delta-9-desaturase) |
| 4 | 211162_x_at | *SCD* | stearoyl-CoA desaturase (delta-9-desaturase) |
| 5 | 202218_s_at | *FADS2* | fatty acid desaturase 2 |
| 6 | 202067_s_at | *LDLR* | low density lipoprotein receptor (familial hypercholesterolemia) |
| 7 | 208964_s_at | *FADS1* | fatty acid desaturase 1 |
| 8 | 201275_at | *FDPS* | farnesyl diphosphate synthase |
| 9 | 201625_s_at | *INSIG1* | insulin induced gene 1 |
| 10 | 202068_s_at | *LDLR* | ow density lipoprotein receptor (familial hypercholesterolemia) |
| 11 | 209608_s_at | *ACAT2* | acetyl-Coenzyme A acetyltransferase 2 (acetoacetyl Coenzyme A thiolase) |
| 12 | 210950_s_at | *FDFT1* | farnesyl-diphosphate farnesyltransferase 1 |
| 13 | 216607_s_at | *CYP51A1* | cytochrome P450, family 51, subfamily A, polypeptide 1 |
| 14 | 212218_s_at | *FASN* | fatty acid synthase |
| 15 | 201790_s_at | *DHCR7* | 7-dehydrocholesterol reductase |
| 16 | 219562_at | *RAB26* | RAB26, member RAS oncogene family |
| 17 | 210130_s_at | *TM7SF2* | transmembrane 7 superfamily member 2 |
| 18 | 208962_s_at | *FADS1* | fatty acid desaturase 1 |
| 19 | 209577_at | *PCYT2* | ohosphate cytidylyltransferase 2, ethanolamine |
| 20 | 202735_at | *EBP* | emopamil binding protein (sterol isomerase) |
| 21 | 211708_s_at | *SCD* | stearoyl-CoA desaturase (delta-9-desaturase) |
| 22 | 201791_s_at | *DHCR7* | 7-dehydrocholesterol reductase |
| 23 | 208926_at | *NEU1* | sialidase 1 (lysosomal sialidase) |
| 24 | 208963_x_at | *FADS1* | fatty acid desaturase 1 |
| 25 | 219181_at | *LIPG* | lipase, endothelial |
| 26 | 209218_at | *SQLE* | squalene epoxidase |
| 27 | 211019_s_at | *LSS* | anosterol synthase (2,3-oxidosqualene-lanosterol cyclase) |
| 28 | 202245_at | *LSS* | lanosterol svnthase (2,3-oxidosqualene-lanosterol cyclase) |
| 29 | 201193_at | *IDH1* | isocitrate dehydrogenase 1 (NADP+), soluble |
| 30 | 201248_s_at | *SREBF2* | sterol regulatory element binding transcription factor 2 |
| 31 | 201626_at | *INSIG1* | insulin induced gene 1 |
| 32 | 220675_s_at | *PNPLA3* | patatin-like phospholipase domain containing 3 |
| 33 | 202275_at | *G6PD* | glucose-6-phosphate dehydrogenase |
| 34 | 202022_at | *ALDOC* | aldolase C, fructose-bisphosphate |
| 35 | 209146_at | *SC4MOL* | sterol-C4-methyl oxidase-like |
| 36 | 205822_s_at | *HMGCS1* | 3-hydroxy-3-methylglutaryl-Coenzyme A synthase 1 (soluble) |
| 37 | 201662_s_at | *ACSL3* | acyl-CoA synthetase long-chain family member 3 |
| 38 | 211070_x_at | *DBI* | diazepam binding inhibitor |
| 39 | 211546_x_at | *SNCA* | synuclein, alpha (non A4 component of amyloid precursor) |
| 40 | 209389_x_at | *DBI* | diazepam binding inhibitor |
| 41 | 213448_at | *MTX1* | CDNA FLJ31688 fis, clone NT2RI2005520 |
| 42 | 200862_at | *DHCR24* | 24-dehydrocholesterol reductase |
| 43 | 204615_x_at | *IDI1* | sopentenyl-diphosphate delta isomerase 1 |
| 44 | 50965_at | *RAB26* | RAB26, member RAS oncogene family |
| 45 | 208647_at | *FDFT1* | farnesyl-diphosphate farnesyltransferase 1 |
| 46 | 204818_at | *HSD17B2* | hydroxysteroid (17-beta) dehydrogenase 2 |
| 47 | 202428_x_at | *DBI* | diazepam binding Inhibitor |
| 48 | 207275_s_at | *ACSL1* | acyl-CoA synthetase long-chain family member 1 |
| 49 | 201127_s_at | *ACLY* | ATP citrate lyase |
| 50 | 206154_at | *RLBP1* | retinaldehyde binding protein 1 |
| 51 | 219194_at | *SEMA4G* | semaphorin 4G |
| 52 | 212276_at | *LPIN1* | ipin 1 |
| 53 | 202314_at | *CYP51A1* | cytochrome P450, family 51, subfamily A, polypeptide 1 |
| 54 | 204467_s_at | *SNCA* | synuclein, alpha (non A4 component of amyloid precursor) |
| 55 | 211391_s_at | *PATZ1* | POZ (BTB) and AT hook containing zinc finger 1 |
| 56 | 215649_s_at | *MVK* | mevalonate kinase (mevalonic aciduria) |
| 57 | 202539_s_at | *HMGCR* | 3-hydroxy-3-methylglutaryl-Coenzyme A reductase |
| 58 | 208452_x_at | *MYO9B* | myosin IXB |
| 59 | 221757_at | *MGC17330* | phosphoinositide-3-kinase interacting protein 1 |
| 60 | 217173_s_at | *LDLR* | ow density lipoprotein receptor (familial hypercholesterolemia) |
| 61 | 201425_at | *ALDH2* | aldehyde dehydrogenase 2 family (mitochondrial) |
| 62 | 201118_at | *PGD* | phosphogluconate dehydrogenase |
| 63 | 208792_s_at | *CLU* | Clusterin |
| 64 | 217775_s_at | *RDH11* | retinol dehydrogenase 11 (all-trans/9-cis/11-cis) |
| 65 | 202752_x_at | *SLC7A8* | solute carrier family 7 (cationic amino acid transporter, y+ system). member 8 |
| 66 | 217436_x_at | *LOC730399* | hypothetical protein LOC730399 |
| 67 | 219636_s_at | *4RMC9* | armadillo repeat containing 9 |
| 68 | 203438_at | *STC2* | stanniocalcin 2 |
| 69 | 206956_at | *BGLAP* | bone gamma-carboxyglutamate (gla) protein (osteocalcin) |
| 70 | 213787_s_at | *EBP* | emopamil binding protein (sterol isomerase) |
| 71 | 215821_x_at | *PSG3* | pregnancy specific beta-1-glycoprotein 3 |
| 72 | 203524_s_at | *MPST* | mercaptopyruvate sulfurtransferase |
| 73 | 204011_at | *SPRY2* | sprouty homolog 2 (Drosophila) |
| 74 | 213167_s_at | *SLC5A3* | solute carrier family 5 (inositol transporters), member 3 |
| 75 | 212816_s_at | *CBS* | cystathionine-beta-synthase |
| 76 | 202436_s_at | *CYP1B1* | cytochrome P450, family 1, subfamily B, polypeptide 1 |
| 77 | 215707_s_at | *PRNP* | prion protein (p27-30) |
| 78 | 208881_x_at | *IDI1* | isopentenyl-diphosphate delta isomerase 1 |
| 79 | 202540_s_at | *HMGCR* | 3-hydroxy-3-methylglutaryl-Coenzyme A reductase |
| 30 | 210372_s_at | *TPD52L1* | tumor protein D52-like 1 |
| 81 | 214326_x_at | *JUND* | jun D proto-oncoqene |
| 82 | 204466_s_at | *SNCA* | synuclein, alpha (non A4 component of amyloid precursor) |
| 83 | 211574_s_at | *CD46* | CD46 molecule, complement regulatory protein |
| 84 | 201427_s_at | *SEPP1* | selenoprotein P, plasma, 1 |
| 85 | 208483_x_at | *KRT33A* | keratin 33A |
| 86 | 201660_at | *ACSL3* | Full-length cDNA clone CS0DF008YJ12 of Fetal brain of Homo sapiens |
| 87 | 208791_at | *CLU* | Clusterin |
| 88 | 201348_at | *GPX3* | glutathione peroxidase 3 (plasma) |
| 89 | 214449_s_at | *RHOQ* | ras homolog gene family, member Q |
| 90 | 211423_s_at | *SC5DL* | sterol-C5-desaturase (ERG3 delta-5-desaturase homolog, S. cerevisiae)-like |
| 91 | 205808_at | *ASPH* | aspartate beta-hydroxylase |
| 92 | 201247_at | --- | sterol regulatory element binding transcription factor 2 |
| 93 | 211284_s_at | *GRN* | Granulin |
| 94 | 219462_at | *TMEM53* | transmembrane protein 53 |
| 95 | 207787_at | *KRT33B* | keratin 33B |
| 96 | 201141_at | *GPNMB* | glycoprotein (transmembrane) nmb |
| 97 | 214283_at | *TMEM97* | transmembrane protein 97 |
| 98 | 214176_s_at | *PBXIP1* | Pre-8-cell leukemia homeobox interacting protein 1 |
| 99 | 213187_x_at | *FTL* | ferritin, light polypeptide |
| 100 | 210519_s_at | *NQO1* | NAD(P)H dehydrogenase, quinone 1 |

**Table 3. Top 20 ranked probe sets of the antipsychotic group profile in the H4 cell line**

| Rank | Probe set | Gene | Description |
|---|---|---|---|
| 1 | 202067_s_at | *LDLR* | low density lipoprotein receptor (familial hypercholesterolemia) |
| 2 | 201626_at | *INSIG1* | insulin induced gene 1 |
| 3 | 208963_x_at | *FADS1* | fatty acid desaturase 1 |
| 4 | 200831_s_at | *SCD* | stearoyl-CoA desaturase (dolta-9-desaturase) |
| 5 | 211019_s_at | *LSS* | lanosterol synthase (2,3-oxidosqualene-lanosterol cyclase) |
| 6 | 216607_s_at | *CYP51A1* | cytochrome P450 family 51, subfamily A, polypeptide 1 |
| 7 | 218169_at | *VAC14* | Vac14 homolog (S cerevisiae) |
| 8 | 201627_s_at | *INSIG1* | insulin induced gene 1 |
| 9 | 200832_s_at | *SCD* | stearoyl-CoA desaturase (delta-9-desaturase) |
| 10 | 209230_s_at | *NUPR1* | nuclear protein 1 |
| 11 | 202068_s_at | *LDLR* | low density lipoprotein receptor (familial hypercholesterolemia) |
| 12 | 201141_at | *GPNMB* | glycoprotein (transmembrane) nmb |
| 13 | 211781_x_at | - - - | - - - |
| 14 | 217173_s_at | *LDLR* | low density lipoprotein receptor (familial hypercholestarolemia) |
| 15 | 221249_s_at | *FAM117A* | family with sequence similarity 117, member A |
| 16 | 213100_at | - - - | unc-5 homolog B (C. elegans) |
| 17 | 201625_s_at | *INSIG1* | insulin induced gene 1 |
| 18 | 203408_s_at | *SATB1* | SATB homeobox 1 |
| 19 | 205168_at | *DDR2* | discoidin domain receptor family, member 2 |
| 20 | 202067_s_at | *LDLR* | low density lipoprotein receptor (familial hypercholesterolemia) |

**Table 4. Ranked list of the bottom 100 probe sets down-regulated by the group of antipsychotic drugs in the ARPE-19 cell line**

| Rank | Probe set | Gene | Description |
|---|---|---|---|
| 1 | 216237_s_at | *MCM5* | minichromosome maintenance complex component 5 |
| 2 | 204822_at | *TTK* | TTK protein kinase |
| 3 | 203145_at | *SPAG5* | sperm associated antigen 5 |
| 4 | 209706_at | *NKX3-1* | NK3 homeobox 1 |
| 5 | 221258_s_at | *KIF18A* | kinesin family member 18A |
| 6 | 205034_at | *CCNE2* | cyclin E2 |
| 7 | 218662_s_at | *NCAPG* | ion-SMC condensin I complex, subunit G |
| 3 | 209709_s_at | *HMMR* | hyaluronan-mediated motility receptor (RHAMM) |
| 3 | 222039_at | *LOC146909* | hypothelical protein LOC146909 |
| 10 | 204146_at | *RAD51AP1* | RAD51 associated protein 1 |
| 11 | 204444_at | *KIF11* | Kinesin family member 11 |
| 12 | 204641_at | *NEK2* | NIMA (never in mitosis gene a)-related kinase 2 |
| 13 | 221685_s_at | *CCDC99* | coiled-coil domain containing 99 |
| 14 | 218219_s_at | *LANCL2* | LanC lantibiotic synthetase component C-like 2 (bacterial) |
| 15 | 213302_at | *PFAS* | phosphoribosylformylglycinamide synthase (FGAR amidotransferase) |
| 16 | 213092_x_at | *DNAJC9* | DnaJ (Hsp40) homolog, subfamily C member 9 |
| 17 | 218585_s_at | *DTL* | denticleless homolog (Drosophila) |
| 18 | 208103_s_at | *ANP32E* | acidic (leucine-rich) nuclear phosphoprotein 32 family, member E |
| 19 | 202952_s_at | *ADAM12* | ADAM metallopeptidase domain 12 (meltrin alpha) |
| 20 | 201664_at | *SMC4* | structural maintenance of chromosomes 4 |
| 21 | 205264_at | *CD3EAP* | CD3e molecule epsilon associated protein |
| 22 | 209773_s_at | *RRM2* | ribonucleotide reductase M2 polypeptide |
| 23 | 201930_at | *MCM6* | minichromosome maintenance complex component 6 |
| 24 | 203895_at | *PLCB4* | phospholipase C beta 4 |
| 25 | 205590_at | *RASGRP1* | RAS guanyl releasing protein 1 (calcium and DAG-regulated) |
| 26 | 211375_s_at | *ILF3* | interleukin enhancer binding factor 3, 90kDa |
| 27 | 205339_at | *STIL* | SCL/TAL1 interrupting locus |
| 28 | 219148_at | *PBK* | PDZ binding kinase |
| 29 | 201292_at | *TOP2A* | topoisomerase (DNA) II alpha 170kDa |
| 30 | 206008_at | *TGM1* | transglutaminase 1 |
| 31 | 209547_s_at | *SF4* | splicing factor 4 |
| 32 | 202668_at | *EFNB2* | ephrin-B2 |
| 33 | 204372_s_at | *KHSRP* | KH-type splicing regulatory protein (FUSE binding protein 2) |
| 34 | 218656_s_at | *LHFP* | lipoma HMGIC fusion partner |
| 35 | 204962_s_at | *CENPA* | centromere protein A |
| 36 | 218550_s_at | *LRRC20* | leucine rich repeat containing 20 |
| 37 | 204141_at | *TUBB2A* | tubulin, beta 2A |
| 38 | 205547_s_at | *TAGLN* | transgelin |
| 39 | 213599_at | *OIP5* | Opa interacting protein 5 |
| 40 | 212242_at | *TUBA1* | tubulin, alpha 4a |
| 41 | 218102_at | *DERA* | 2-deoxyribose-5-phosphate aldolase homolog (C elegans) |
| 42 | 211933_s_at | *HNRPA3P1* | heterogeneous nuclear ribonucleoprotein A3 pseudogene 1 |
| 43 | 202397_at | *NUTF2* | nuclear transport factor 2 |
| 44 | 204092_s_at | *AURKA* | aurora kinase A |
| 45 | 32723_at | *CSTF1* | cleavage stimulation factor 3' pre-RNA, subunit 1, 50kDa |
| 46 | 209817_at | *PPP3CR* | protein phosphatase 3 (formerly 2B), catalytic subunit beta isoform |
| 47 | 200040_at | *KHDRBS1* | KH domain containing, RNA binding, signal transduction associated 1 |
| 48 | 203074_at | *ANXA8* | annexin A8-like 2 /// annexin A8 /// annexin A8-like 1 |
| 49 | 209291_at | *ID4* | inhibitor ot DNA binding 4, dominant negative helix-loop-protein protein |
| 50 | 201609_x_at | *ICMT* | isoprenylcysteine carboxyl methyltransferase |
| 51 | 201897_s_at | *CKS1B* | CDC28 protein kinase regulatory subunit 1B |
| 52 | 201013_s_at | *PAICS* | phosphoribosylaminoimidazole carboxylase |
| 53 | 218883_s_at | *MLF1IP* | MLF1 interacting protein |
| 54 | 219493_at | *SHCBP1* | SHC SH2-domain binding protein 1 |
| 55 | 209642_at | *BUB1* | BUB1 budding uninhibited by benzimidazoles 1 homolog yeast) |
| 56 | 219787_s_at | *ECT2* | epithelial cell transforming sequence 2 oncogene |
| 57 | 206364_at | *KIF14* | kinesin family member 14 |
| 58 | 206055_s_at | *SNRPA1* | small nuclear ribonucleoprotein polypeptide A' |
| 59 | 221381_s_at | *MORF4L1* | mortality factor 4 like 1 /// mortality factor 4 |
| 60 | 208694_at | *PRKDC* | protein kinase DNA-activated, catalytic polypeptide |
| 61 | 220651_s_at | *MCM10* | minichromosome maintenance complex component 10 |
| 62 | 201755_at | *MCM5* | minichromosome maintenance complex component 5 |
| 33 | 213253_at | *SMC2* | structural maintenance of chromosomes 2 |
| 34 | 203022_at | *RNASEH2A* | ribonuclease H2 subunit A |
| 65 | 212846_at | *KIAA0179* | ribosomal RNA processing 1 homolog B (S. cerevisiae) |
| 66 | 203359_s_at | *MYCBP* | c-myc binding protein |
| 67 | 203276_at | *LMNB1* | a min B1 |
| 68 | 209314_s_at | *HBS1L* | HBS1 like (S. cerevisiae) |
| 69 | 210766_s_at | *CSE1L* | CSE1 chromosome segregation 1-like (yeast) |
| 70 | 205807_s_at | *TUFT1* | tuftelin 1 |
| 71 | 219222_at | *RBKS* | ribokinase |
| 72 | 210559_s_at | *CDC2* | cell division cycle 2. G1 to S and G2 to M |
| 73 | 222047_s_at | *ARS2* | ARS2 protein |
| 74 | 206102_at | *GINS1* | GINS complex subunit 1 (Psf1 homolog) |
| 75 | 204728_s_at | *WDHD1* | WD repeat and HMG box DNA binding protein 1 |
| 76 | 204023_at | *RFC4* | replication factor C (activator 1) 4, 37kDa |
| 77 | 211671_s_at | *NR3C1* | nuclear receptor subfamily 3 group C, member 1 (glucocorticoid receptor) |
| 78 | 214741_at | *ZNF131* | zinc finger protein 131 |
| 79 | 218663_at | *NCAPG* | non-SMC condensin I complex, subunit G |
| 80 | 204127_at | *RFC3* | replication factor C (activator 1) 3, 38kDa |
| 81 | 202925_s_at | *PLAGL2* | pleiomorphic adenoma gene-like 2 |
| 82 | 201453_x_at | *RHEB* | Ras homolog enriched in brain |
| 83 | 221634_at | *RPL23AP7* | ribosomal protein L23a pseudogene 7 |
| 94 | 203505_at | *ABCA1* | ATP-binding cassette, sub-family A (ABC1), member 1 |
| 85 | 200041_s_at | *BAT1* | HLA-B associated transcript 1 |
| 86 | 204887_s_at | *PLK4* | polo-like kinase 4 (Drosophila) |
| 87 | 221521_s_at | *GINS2* | GINS complex subunit 2 (Psf2 homolog) |
| 88 | 202937_x_at | *CTA-126B4* | CGI-96 protein |
| 89 | 222077_s_at | *RACGAP1* | Rac GTPase activating protein 1 |
| 90 | 221872_at | *RARRES1* | retinoic acid receptor responder (tazarotene induced) 1 |
| 91 | 209909_s_at | --- | transforming growth factor, beta 2 |
| 92 | 221845_s_at | *CLPB* | ClpB caseinolytic peptidase B homolog (E coli) |
| 93 | 213790_at | --- | ADAM metallopeptidase domain 12 (meltrin alpha) |
| 94 | 218980_at | *FHOD3* | formin homology 2 domain containing 3 |
| 95 | 212037_at | *PNN* | pinin, desmosome associated protein |
| 96 | 212767_at | *MTG1* | mitochondrial GTPase 1 homolog (S. cerevisiae) |
| 97 | 219920_s_at | *GMPPB* | GDP-mannose pyrophosphorylase B |
| 98 | 204531_s_at | *BRCA1* | breast cancer 1, early onset |
| 99 | 204162_at | *KNTC2* | NDC80 homolog, kinetochore complex component (S cerevisiae) |
| 100 | 218340_s_at | *UBE1L2* | ubiquitin-activating enzyme E1-like 2 |

**Table 5. Top 20 ranked probe sets of the selective estrogen receptor modulators in the ARPE-19 cell line**

| Rank | Probe set | Gene | Description |
|---|---|---|---|
| 1 | 200831_s_at | *SCD* | stearoyl-CoA desaturase (delta-9-desaturase) |
| 2 | 201625_s_at | *INSIG1* | insulin induced gene 1 |
| 3 | 201626_at | *INSIG1* | insulin induced gene 1 |
| 4 | 202068_s_at | *LDLR* | low density lipoprotein receptor |
| 5 | 211162_x_at | *SCD* | stearoyl-CoA desaturase (delta-9-desaturase) |
| 6 | 211708_s_at | *SCD* | stearoyl-CoA desaturase (delta-9-desaturase) |
| 7 | 200832_s_at | *SCD* | stearoyl-CoA desaturase (delta-9-desaturase) |
| 8 | 217173_s_at | *LDLR* | low density lipoprotein receptor |
| 9 | 201275_at | *FDPS* | farnesyl diphosphate synthase |
| 10 | 201791_s_at | *DHCR7* | 7-dehydrocholesterol reductase |
| 11 | 212218_s_at | *FASN* | fatty acid synthase |
| 12 | 210950_s_at | *FDFT1* | farnesyl-diphosphate farnesyltransferase 1 |
| 13 | 203027_s_at | *MVD* | mevalonate (diphospho) decarboxylase |
| 14 | 201790_s_at | *DHCR7* | 7-dehydrocholesterol reductase |
| 15 | 201627_s_at | *INSIG1* | insulin induced gene 1 |
| 16 | 202245_at | *LSS* | lanosterol synthase |
| 17 | 2046155_x_at | *IDI1* | isopentenyl-diphosphate delta isomerase 1 |
| 18 | 211018_at | *LSS* | lanosterol synthase |
| 19 | 202218_s_at | *FADS2* | fatty acid desaturase 2 |
| 20 | 216607_s_at | *CYP51A1* | cytochrome P450, family 51, subfamily A. polypeptide 1 |

**Table 6. Comparison of the antipsychotic gene expression signature across a library of compounds**

| Class | Subclass | N | KS | Amplitude |
|---|---|---|---|---|
| central nervous system agent | antipsychotic | 18 | 0.999 | 0.56 |
| SERM | | 3 | 0.997 | 1.02 |
| central nervous system agent | antidepressant | 20 | 0.898 | 0.31 |
| cardiovascular agent | antihyperlipidemic | 8 | 0.768 | 0.17 |
| respiratory agent | histamine antagonist | 13 | 0.728 | 0.22 |
| | | | | |
| cardiovascular agent | antiarrhythmic | 8 | 0.725 | 0.2 |
| dermatological agent | antifungal | 12 | 0.614 | 0.18 |
| cardiovascular agent | calcium channel blocker | 9 | 0.491 | 0.13 |
| | | | | |
| respiratory agent | antiasthma | 16 | 0.462 | 0.03 |
| anti infective agent | antimalarial | 5 | 0.459 | 0.15 |
| genitourinary agent | female reproductive agent | 12 | 0.451 | 0 |
| | | | | |
| dermatological agent | anesthetic local | 6 | 0.4 | 0.11 |
| endocrine metabolic agent | antidiabetic | 10 | 0.397 | 0.02 |
| anti infective agent | antiviral | 7 | 0.352 | 0.05 |
| gastrointestinal agent | cholinergic | 8 | 0.333 | 0.03 |
| cardiovascular agent | adrenergic blocker | 12 | 0.329 | 0.03 |
| nutritive agent | vitamin class | 5 | 0.314 | 0.02 |
| anti infective agent | antitubercular | 8 | 0.31 | 0.01 |
| anti infective agent | antifungal | 11 | 0.297 | 0.07 |
| endocrine metabolic agent | calcium regulator | 5 | 0.274 | 0.03 |
| cardiovascular agent | antihypertensive | 19 | 0.265 | -0.05 |
| endocrine metabolic agent | corticosteroid | 7 | 0.262 | 0.05 |
| dermatological agent | anti inflammatory | 9 | 0.246 | 0.02 |
| genitourinary agent | renal urologic agent | 8 | 0.243 | 0.08 |
| anesthetic agent | anesthetic local | 6 | 0.22 | -0.01 |
| nasal agent | anti inflammatory | 5 | 0.203 | -0.02 |
| central nervous system agent | antiparkinsonian | 8 | 0.202 | -0.02 |
| endocrine metabolic agent | estrogen | 5 | 0.199 | 0 |
| ophthalmologic agent | antiglaucoma | 11 | 0.197 | -0.01 |
| respiratory agent | cold cough agent | 6 | 0.193 | 0 |
| musculoskeletal agent | skeletal muscle relaxant | 8 | 0.178 | 0.01 |
| | | | | |
| cardiovascular agent | diuretic | 17 | 0.163 | 0.01 |
| central nervous system agent | analgesic | 22 | 0.148 | 0 |
| nutritive agent | dietary supplement | 11 | 0.087 | -0.02 |
| central nervous system agent | anticonvulsant | 13 | -0.116 | -0.05 |
| nasal agent | decongestant | 6 | -0.192 | -0.05 |
| nutritive agent | mineral supplement | 8 | -0.205 | -0.02 |
| ophthalmologic agent | antibiotic | 9 | -0.206 | -0.18 |
| gastrointestinal agent | antiulcer | 9 | -0.208 | -0.08 |
| musculoskeletal agent | neuromuscular blocker | 6 | -0.222 | -0.04 |
| | | | | |
| cardiovascular agent | vasopressor | 8 | -0.225 | -0.08 |
| cardiovascular agent | vasodilator | 6 | -0.24 | -0.08 |
| antineoplastic agent | antimetabolite | 8 | -0.265 | -0.06 |
| anti infective agent | antibiotic | 70 | -0.334 | -0.07 |
| dermatological agent | antibacterial | 9 | -0.334 | -0.19 |
| antineoplastic agent | mitotic inhibitor | 5 | -0.343 | -0.03 |
| antineoplastic agent | alkylating agent | 13 | -0.367 | -0.12 |
| dermatological agent | antiacne | 9 | -0.398 | -0.09 |

Thus, from the preceding description and examples, it will be appreciated that one illustrative aspect of this invention is a method of screening for compounds, including small molecules and macromolecules such as polypeptides and proteins, having a given activity that comprises identifying a molecular signature for compounds known to have the given activity and then screening compounds not known to have the given activity to identify compounds among the unknowns that have that molecular signature and therefore have or are likely to have the given activity. The molecular signature is a ranking of genes that are upregulated and/or downregulated in response to treatment with a compound having a given pharmacologic activity. The molecular signature can also be a scoring based on absolute increases or decreases in gene expression but as illustrated hereinabove, it is sufficient to identify a signature based on ranking in order of percent change in expression. Ranking can be based on amplitude which, as discussed above, describes the level of expression in treated cells relative to untreated cells. Generally, an amplitude of about 0.4 is regarded as important and therefore can be used to define a threshold for upregulation or downregulation. The threshold can be set at other amplitudes, e.g., about 0.3, about 0.5, about 0.6, about 0.7, and about 0.8. Change in expression (upregulation or downregulation) can be measured at the level of transcription (mRNA), translalion (gene product), or gene product activity (biochemical changes, e.g., changes in tissue lipids.)

The molecular signature of a class of therapeutics becomes more refined as more agents of the same class are tested for effects on gene regulation. In some cases, it may be necessary to discard outliers using standard statistical analyses. The molecular signature can also vary depending upon the choice and number of probes and of the cells or cell lines that are treated. Nevertheless, by testing a sufficient number of compounds in one or more cell types with a sufficient quantity and diversity of probes, it is possible to identify a central theme. For example, in the case of antipsychotics, regulation of genes involved in lipid homeostasis is a theme that underpins the molecular signature for this class of drugs.

It is preferable to use probes for a large number of genes, e.g., at least about 1000 genes, or at least about 5000 genes, or at least about 10000 genes. Use of multiple probes for each gene can refine the signature to a further level of detail.

The cells used can be derived from any animal, including human or other mammalian cells. But, other cell types, such as Saccharomyces sp., can also be used.

In accordance with one illustrative embodiment of the invention, compounds not known to have antipsychotic or anti-schizophrenic activity can be assayed for such activity by screening for compounds that cause altered expression of genes involved in lipid homeostasis. Without intending to be bound to this mechanism, such compounds can modulate the ratio of polyunsaturated to saturated fatty acids and/or cholesterol content and that thereby alter the fluidity of cell membranes of neurons and supporting cells, resulting in changes in neuronal connectivity.

Specifically, for example, compounds whose molecular signature is an increase in expression in any one or more of the genes listed in Table 2 are selected as compounds that are likely to have antipsychotic and/or anti-schizophrenic activity. There are 76 genes identified in Table 2. In alphabetical order, these are:
*ACAT2*
*ACLY*
*ACSL1*
*ACSL3*
*ALDH2*
*ALDOC*
*ARMC9*
*ASPH*
*BGLAP*
*CBS*
*CD46*
*CLU*
*CYP1B1*
*CYP51A1*
*DBI*
*DHCR24*
*DHCR7*
*EBP*
*FADS1*
*FADS2*
*FASN*
*FDFT1*
*FDPS*
*FTL*
*G6PD*
*GPNMB*
*GPX3*
*GRN*
*HMGCR*
*HMGCS1*
*HSD17B2*
*IDH1*
*IDI1*
*INSIG1*
*JUND*
*KRT33A*
*KRT33B*
*LDLR*
*LIPG*
*LOC730399*
*LPIN1*
*LSS*
*MGC17330*
*MPST*
*MTX1*
*MVK*
*MYO9B*
*NEU1*
*NQO1*
*PATZ1*
*PBXIP1*
*PCYT2*
*PGD*
*PNPLA3*
*PRNP*
*PSG3*
*RAB26*
*RDH11*
*RHOQ*
*RLBP1*
*SC4MOL*
*SC5DL*
*SCD*
*SEMA4G*
*SEPP1*
*SLC5A3*
*SLC7A8*
*SNCA*
*SPRY2*
*SQLE*
*SREBF2*
*STC2*
*TM7SF2*
*TMEM53*
*TMEM97*
*TPD52L1*

In some embodiments, the molecular signature comprises upregulation of at least 2 of these genes, e.g., an amplitude of about 0.4 or greater, or of about 0.5 or greater, or of about 0.6 or greater, or of about 0.7 or greater. In other embodiments, at least 5 or more, 10 or more, 20 or more, 30 ore more, 40 or more, 50 or more, 60 or more, 70 or more, or all 76 genes are upregulated.

In another illustrative embodiment, compounds whose molecular signature comprises upregulation of one or more, e.g., 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more or all 19 of the genes that appear in both Tables 1 and 3, i.e.: INSIG1, SCD, FADS2, LDLR, FADS1, FDPS, ACAT2, FDFT1, CYP51A1, FASN, DHCR7, RAB26, TM7SF2, SATB1, FAM117A, GPNMB, NUPR1, VAC14, and LSS, are selected as compounds that are likely to have antipsychotic and/or anti-schizophrenic activity.

In another illustrative embodiment of the invention, compounds can be assayed for antipsychotic and/or anti-schizophrenic activity by screening for compounds that cause an increase in expression in any one or more, e.g., 3 or more, 4 or more, 5 or more, 6 or more, 7 or more, 8 or more, 9 or more, 10 or more, 11 or more, 12 or more, or all 13 of the genes in Table 1, i.e.: INSIG1, SCD, FADS2, LDLR, PADS1, FDPS, ACAT2, FDFT1, CYP51A1, FASN, DHCR7, RAB26, and TM7SF2.

In other illustrative embodiments, compounds are selected based on upregulation of one or more of the genes listed in Table 3, i.e.: LDLR, INSIG1, FADS1, SCD, LSS, CYP51A1, VAC 14, NUPR1, GPNMB, FAM117A, SATB1 and DDR2, or in both Tables 1 and 3, i.e.: INSIG1, SCD, LDLR, FADS1, and CYP51A1.

In other illustrative embodiments, ranking is relied upon for a more refined molecular signature. Thus, one could simply make a list of genes that are upregulated and determine if one or more of the genes recited above are upregulated or one could rank order such list based on amplitude and compare that to the gene rankings obtained with a given class of drugs, e.g., as shown in in Table 1 (or Table 2 or 3.) The more similar the molecular signature by ranking for a given drug is to the molecular signature by ranking of known antipsychotics, as illustrated above, the greater the likelihood is that the compound will have antipsychotic activity. Thus, in an illustrative embodiment of this invention, the molecular signature is defined not merely as which genes are upregulated or downregulated but also by ranking based on the extent of change in expression, e.g., by amplitude, such that a given gene that is upregulated to a greater relative extent than a second gene is ranked higher than the second gene. So, a gene whose expression is trebled would be ranked higher than a gene whose expression is doubled, regardless of the absolute quantity of mRNA or protein transcribed or expressed.

So, for example, with reference to Table 1, if a drug causes increased expression of the INSIG1 and SCD genes, it can be regarded as having a molecular signature that is similar to the molecular signature of an antipsychotic. However, if the change in expression of the INSIG1 gene (probe set 201627_s_at) is greater than that of the SCD gene (probe set 200831_s_at), then, in accordance with this illustrative embodiment, it has a molecular signature by ranking that is similar to the molecular signature of an antipsychotic. So, in accordance with this embodiment, a compound not known to have antipsychotic activity is selected as having or being likely to have antipsychotic activity based on whether or not it has a molecular signature by tanking that is similar to the molecular signature of an antipsychotic, such as the molecular signature defined in Table 1 or other of the tables below.

Relative expression of any two or more genes can be used but, as discussed above, the molecular signature becomes more refined when greater numbers of genes are compared. Drugs with antipsychotic activity are useful in the treatment of psychotic and other mental or emotional disorders, e.g., bipolar disorder, schizophrenia, mania, and depression. Thus, an illustrative embodiment of this invention comprises use of agents identified in a screen such as described above in treating psychotic or other disorders that are amenable to treatment with an antipsychotic, i.e., disorders for which treatment with an antipsychotic agent is indicated. Use of known typical or atypical antipsychotics are not embraced within the scope of this aspect of the invention. Thus, another illustrative embodiment is a method of preventing or treating a disorder that is amenable to treatment with an antipsychotic that comprises internally administering to a patient an agent, other than a known typical or atypical antipsychotic, wherein the agent has a molecular signature similar to an antipsychotic molecular signatures such as is described above.

In another illustrative embodiment of the invention, monitoring of expression of genes that alter lipid homeostasis is used as a biomarker for antipsychotic activity in a subject, e.g., a human patient. As discussed above, expression can be monitored in a variety of ways, including by assaying for transcription, for translation, or biochemically. Detection of upregulation of genes as described above indicates that the drug is having the desired effect on the subject and thus can indicate efficacy even in the absence of a detectable clinical change. With such information, a physician can make earlier decisions about changing the treatment regiment such as by increasing or decreasing dose or by changing to a supplemental or alternative therapy. Monitoring can be accomplished by assaying samples of bodily tissue, e.g., following removal from the body, and can make use of any one or more of the signatures described above (or alternative signatures generated using different cells and/or different probes.)

As described above, the above data indicate that the SERMs, tamoxifen, raloxifene, and clomiphene also alter lipid homeostasis. These SERMs have a similar molecular signature to antipsychotics.

Thus, in a related illustrative embodiment of this invention, the molecular signatures described above are used to identify compounds that are likely to have selective estrogen receptor modulatory activity, to monitor subjects being treated with a SERM, and to treat patients in need of estrogen receptor modulation.

The foregoing description of various aspects of the invention has been presented for purposes of illustration and description. It is not intended to be exhaustive or to limit the invention to the precise form disclosed, and obviously, many modifications and variations are possible. Such modifications and variations that may be apparent to a person skilled in the art are intended to be included within the scope of the invention as defined by the accompanying claims.

### Reference List

Baptista,T., de Baptista,E.A., Hernandez,L., Altemus,M., and Weiss,S.R., 1997. Tamoxifen prevents sulpiride-induced weight gain in female rats. Pharmacol Biochem Behav. 57, 215-222.
Carver,J.D., Benford,V.J., Han,B., and Cantor,A.B., 2001. The relationship between age and the fatty acid composition of cerebral cortex and erythrocytes in human subjects. Brain Res Bull. 56, 79-85.
Chen,M.L. and Chen,C.H., 2005. Microarray analysis of differentially expressed genes in rat frontal cortex under chronic risperidone treatment. Neuropsychopharmacology. 30, 268-277.
Cheng,M.C., Liao,D.L., Hsiung,C.A., Chen,C.Y., Liao,Y.C., and Chen,C.H., 2008. Chronic treatment with aripiprazole induces differential gene expression in the rat frontal cortex. Int J Neuropsychopharmacol. 11, 207-216.
Christensen,O. and Christensen,E., 1988. Fat consumption and schizophrenia. Acta Psychiatr Scand. 78, 587-591.
Duncan,C.E., Chetcuti,A.F., and Schofield,P.R., 2008. Coregulation of genes in the mouse brain following treatment with clozapine, haloperidol, or olanzapine implicates altered potassium channel subunit expression in the mechanism of antipsychotic drug action. Psychiatr Genet. 18, 226-239.
Emsley,R., Myburgh,C., Oosthuizen,P., and van Rensburg,S.J., 2002. Randomized, placebo-controlled study of ethyl-eicosapentaenoic acid as supplemental treatment in schizophrenia. Am J Psychiatry. 159, 1596-1598.
Emsley.R., Rabinowitz,J., and Medori,R., 2006. Time course for antipsychotic treatment response in first-episode schizophrenia. Am J Psychiatry. 163, 743-745.
Fatemi,S.H., Reutiman,T.J., Folsom,T.D., Bell,C., Nos,L., Fried,P., Pearce,D.A., Singh,S., Siderovski,D.P., Willard,F.S., and Fukuda,M., 2006. Chronic olanzapine treatment causes differential expression of genes in frontal cortex of rats as revealed by DNA microarray technique. Neuropsychopharmacology. 31, 1888-1899.
Ferno,J., Skrede,S., Vik-Mo,A.O., Havik,B., and Steen,V.M., 2006. Drug-induced activation of SREBP-controlled lipogenic gene expression in CNS-related cell lines: marked differences between various antipsychotic drugs. BMC Neurosci. 7, 69.
Fornaro,P., Calabria,G., Corallo,G., and Picotti,G.B., 2002. Pathogenesis of degenerative retinopathies induced by thioridazine and other antipsychotics: a dopamine hypothesis. Doc Ophthalmol. 105, 41-49.
Freedman,R., Leonard,S., Olincy,A., Kaufmann,C.A., Malaspina,D., Cloninger,C.R., Svrakic,D., Faraone,S.V., and Tsuang,M.T., 2001. Evidence for the multigenic inheritance of schizophrenia. Am J Med Genet. 105, 794-800.
Gattaz,W.F., Schmitt,A., and Maras,A., 1995. Increased platelet phospholipase A2 activity in schizophrenia. Schizophr Res. 16, 1-6.
Goff,D.C. and Coyle,J.T., 2001. The emerging role of glutamate in the pathophysiology and treatment of schizophrenia. Am J Psychiatry. 158, 1367-1377.
Grigoriadis,S. and Seeman,M.V., 2002. The role of estrogen in schizophrenia: implications for schizophrenia practice guidelines for women. Can J Psychiatry. 47, 437-442.
Hanson,D.R. and Gottesman,I.I., 2005. Theories of schizophrenia: a genetic-inflammatory-vascular synthesis. BMC Med Genet. 6, 7.
Harrop,C. and Trower,P., 2001. Why does schizophrenia develop at late adolescence? Clin Psychol Rev. 2.1, 241-265.
Hibbeln,J.R., 1998. Fish consumption and major depression. Lancet. 351, 1213.
Horrobin,D.F., 1998. The membrane phospholipid hypothesis as a biochemical basis for the neurodevelopmental concept of schizophrenia. Schizophr Res. 30, 193-208.
Horrobin,D,F., Manku,M.S., Hillman,H., Iain,A., and Glen,M., 1991. Fatty acid levels in the brains of schizophrenics and normal controls. Biol Psychiatry. 30, 795-805.
Hulbert,A.J., 2007. Membrane fatty acids as pacemakers of animal metabolism. Lipids. 42, 811-819.
Kaddurah-Daouk,R., McEvoy,J., Baillie,R.A., Lee,D., Yao,J.K., Doraiswamy,P.M., and Krishnan,K.R., 2007. Metabolomic mapping of atypical antipsychotic effects in schizophrenia. Mol Psychiatry. 12, 934-945.
Komoroski,R.A., Pearce,J.M., Griffin,W.S., Mrak,R.E., Omori,M., and Karson,C.N., 2001. Phospholipid abnormalities in postmortem schizophrenic brains detected by 31P nuclear magnetic resonance spectroscopy: a preliminary study. Psychiatry Res. 106, 171-180.
Kulkarni,J., Garland,K.A., Scaffidi,A., Headey,B., Anderson,R., de Castella,A., Fitzgerald,P., and Davis,S.R., 2006. A pilot study of hormone modulation as a new treatment for mania in women with bipolar affective disorder. Psychoneuroendocrinology. 31, 543-547.
Kulkarni,J., Gurvich,C., Gilbert,H., Mehmedbegovic,F., Mu,L., Marston,N., Gavrilidis,E., and de Castella,A., 2008. Hormone modulation: a novel therapeutic approach for women with severe mental illness. Aust N Z J Psychiatry. 42, 83-88.
Lamb,J., Crawford,E.D., Peck,D., Modell,J.W., Blat,I.C., Wrobel,M.J., Lerner,J., Brunet,J.P., Subramanian,A., Ross,K.N., Reich,M., Hieronymus,H., Wei,G., Armstrong,S.A., Haggarty,S.J., Clemons,P.A., Wei,R., Carr,S.A., LanderE.S., and Golub,T.R., 2006. The Connectivity Map: using gene-expression signatures to connect small molecules, genes, and disease. Science. 313, 1929-1935.
Lavedan,C., Volpi,S., Polymeropoulos,M.H., and Wolfgang,C.D., 2008. Effect of a ciliary neurotrophic factor polymorphism on schizophrenia symptom improvement in an iloperidone clinical trial. Pharmacogenomics. 9, 289-301.
Lieberman,J.A., Stroup,T.S., McEvoy,J.P., Swartz,M.S., Rosenheck,R.A., Perkins,D.O., Keefe,R.S., Davis,S.M., Davis,C.E., Lebowitz,B.D., Severe,J., and Hsiao,J.K., 2005. Effectiveness of Antipsychotic Drugs in Patients with Chronic Schizophrenia. N Engl J Med. 353, 1209-1223.
Lomax,J., 2005. Get ready to GO! A biologist's guide to the Gene Ontology. Brief Bioinform. 6, 298-304.
Lum,P.Y., Armour,C.D., Stepaniants,S.B., Cavet,G., Wolf,M.K., Butler,J.S., Hinshaw,J.C., Garnier,P., Prestwich,G.D., Leonardson,A., Garrett-Engele,P., Rush,C.M., Bard,M., Schimmack,G., Phillips,J.W., Roberts,C.J., and Shoemaker,D.D., 2004. Discovering modes of action for therapeutic compounds using a genome-wide screen of yeast heterozygotes. Cell. 116, 121-137.
Maida,M.E., Hurley,S.D., Daeschner,J.A., Moore,A.H., and O'Banion,M.K., 2006. Cytosolic prostaglandin E2 synthase (cPGES) expression is decreased in discrete cortical regions in psychiatric disease. Brain Res. 1103, 164-172.
McNamara,R.K., Jandacek,R., Rider,T., Tso,P., Hahn,C.G., Richtand,N.M., and Stanford,K.E., 2007. Abnormalities in the fatty acid composition of the postmortem orbitofrontal cortex of schizophrenic patients: gender differences and partial normalization with antipsychotic medications. Schizophr Res. 91, 37-50.
Nasrallah,H.A., 1993. Neurodevelopmental pathogenesis of schizophrenia. Psychiatr Clin North Am. 16, 269-280.
Newcomer,J.W., 2007. Antipsychotic medications: metabolic and cardiovascular risk. J Clin Psychiatry. 68 Suppl 4, 8-13.
Newman,J.C, and Weiner,A.M., 2005. L2L: a simple tool for discovering the hidden significance in microarray expression data. Genome Biol. 6, R81.
Ntambi,J.M., 1999. Regulation of stearoyl-CoA desaturase by polyunsaturated fatty acids and cholesterol. J Lipid Res. 40, 1549-1558.
Pect,M., 2003. Eicosapentaenoic acid in the treatment of schizophrenia and depression: rationale and preliminary double-blind clinical trial results. Prostaglandins Leukot Essent Fatty Acids. 69, 477-485.
Power,R.F., Mani,S.K., Codina,J., Conneely,O.M., and O'Malley,B.W., 1991. Dopaminergic and ligand-independent activation of steroid hormone receptors. Science. 254, 1636-1639.
Puri,B.K., Richardson,A.J., Horrobin,D.F., Easton,T., Saeed,N., OatridgE,A., Hajnal,J.V., and Bydder,G.M., 2000. Eicosapentaenoic acid treatment in schizophrenia associated with symptom remission, normalisation of blood fatty acids, reduced neuronal membrane phospholipid turnover and structural brain changes. Int J Clin Pract. 54, 57-63.
Raedler,T.J., Bymaster,F.P., Tandon,R., Copolov,D., and Dean,B., 2007. Towards a muscarinic hypothesis of schizophrenia. Mol Psychiatry. 12, 232-246.
Reddy,R.D., Keshavan,M.S., and Yao,J.K., 2004. Reduced red blood cell membrane essential polyunsaturated fatty acids in first episode schizophrenia at neuroleptic-naive baseline. Schizophr Bull. 30, 901-911.
Reis,L.C. and Hibbeln,J.R., 2006. Cultural symbolism of fish and the psychotropic properties of omega-3 fatty acids. Prostaglandins Leukot Essent Fatty Acids. 75, 227-236.
Richardson AJ., 2003. The role of omega 3 fatty acids in behaviour, cognition and mood. Scandinavian Journal of Nutrition. 47, 92-98.
Richardson,A.J., Easton,T., Gruzelier,J.H., and Puri,B.K., 1999. Laterality changes accompanying symptom remission in schizophrenia following treatment with eicosapentaenoic acid. Int J Psychophysiol. 34, 333-339.
Rudin,D.O., 1981. The major psychoses and neuroses as omega-3 essential fatty acid deficiency syndrome: substrate pellagra. Biol Psychiatry. 16, 837-850.
Rudin,D.O., 1982. The dominant diseases of modernized societies as omega-3 essential fatty acid deficiency syndrome: substrate beriberi. Med Hypotheses. 8, 17-47.
Ryan,M.C., Collins,P., and Thakore,J.H., 2003. Impaired fasting glucose tolerance in first-episode, drug-naive patients with schizophrenia. Am J Psychiatry. 160, 284-289.
Schmitt,A., Wilczek,K., Blennow,K., Maras,A., Jatzko,A., Petroianu,G., Braus,D.F., and Gattaz,W.F., 2004. Altered thalamic membrane phospholipids in schizophrenia: a postmortem study. Biol Psychiatry. 56, 41-45.
Sivrioglu,E.Y., Kirli,S., Sipahioglu,D., Gursoy,B., and Sarandol,E., 2007. The impact of omega-3 fatty acids, vitamins E and C supplementation on treatment outcome and side effects in schizophrenia patients treated with haloperidol: an open-label pilot study. Prog Neuropsychopharmacol Biol Psychiatry. 31, 1493-1499.
Sleeman,M.W., Garcia,K., Liu,R., Murray,J.D., Malinova,L., Moncrieffe,M., Yancopoulos,G.D., and Wiegand,S.J., 2003. Ciliary neurotrophic factor improves diabetic parameters and hepatic steatosis and increases basal metabolic rate in db/db mice. Proc Natl Acad Sci U S A. 100, 14297-14302.
Smesny,S., Kinder,D., Willhardt,I., Rosburg,T., Lasch,J., Berger,G., and Sauer,H., 2005. Increased calcium-independent phospholipase A2 activity in first but not in multiepisode chronic schizophrenia. Biol Psychiatry. 57, 399-405.
Thakore,J.H., Mann,J.N., Vlahos,I., Martin,A., and Reznek,R., 2002. Increased visceral fat distribution in drug-naive and drug-free patients with schizophrenia. Int J Obes Relat Metab Disord. 26, 137-141.
Thomas,E.A., George,R.C., Danielson,P.E., Nelson,P.A., Warren,A.J., Lo,D., and Sutcliffe,J.G., 2003. Antipsychotic drug treatment alters expression of mRNAs encoding lipid metabolism-related proteins. Mol Psychiatry. 8, 983-93, 950.
Toda,M. and Abi-Dargham,A., 2007. Dopamine hypothesis of schizophrenia: making sense of it all. Curr Psychiatry Rep. 9, 329-336.
Tsuang,M.T., Bar,J.L., Stone,W.S., and Faraone,S.V., 2004. Gene-environment interactions in mental disorders. World Psychiatry. 3, 73-83.
Van Os, J. and Sham, P. The Epidemiology of Schizophrenia. Murray, R. M., Jones, P. B., Susser, E., Van Os, J., and Cannon, M. 235-254. 2003. Cambridge University Press.
Ref Type: Serial (Book,Monograph)
Vik-Mo,A.O., Birkenaes,A.B., Ferno,J., Jonsdottir,H., Andreassen,O.A., and Steen,V.M., 2008. Increased expression of lipid biosynthesis genes in peripheral blood cells of olanzapine-treated patients. Int J Neuropsychopharmacol. 1-6.
Wang,H.H., Afdhal,N.H., and Wang,D.Q., 2006. Overexpression of estrogen receptor alpha increases hepatic cholesterogenesis, leading to biliary bypersecretion in mice. J Lipid Res. 47, 778-786.
Weickert,C.S., Miranda-Angulo,A.L., Wong,J., Perlman,W.R., Ward,S.E., Radhakrishna,V., Straub,R.E., Weinberger,D.R., and Kleinman,J.E., 2008. Variants in the estrogen receptor alpha gene and its mRNA contribute to risk for schizophrenia. Hum Mol Genet. 17, 2293-2309.
Yao,J.K., Leonard,S., and Reddy,R.D., 2000. Membrane phospholipid abnormalities in postmortem brains from schizophrenic patients. Schizophr Res. 42, 7-17.
Yao,J.K., Magan,S., Sonel,A.F., Gurklis,J.A., Sanders,R., and Reddy,R.D., 2004. Effects of omega-3 fatty acid on platelet serotonin responsivity in patients with schizophrenia. Prostaglandins Leukot Essent Fatty Acids. 71, 171-176.
Yildiz,A., Guleryuz,S., Ankerst,D.P., Ongur,D., and Renshaw,P.F., 2008. Protein kinase C inhibition in the treatment of mania: a double-blind, placebo-controlled trial of tamoxifen. Arch Gen Psychiatry. 65, 255-263.
Zarate,C.A., Jr., Singh,J.B., Carlson,P.J., Quiroz,J., Jolkovsky,L., Luckenbaugh,D.A., and Manji,H.K., 2007. Efficacy of a protein kinase C inhibitor (tamoxifen) in the treatment of acute mania: a pilot study. Bipolar Disord. 9, 561-570.

## Claims

1. A method of screening agents for antipsychotic activity, the method comprising:
contacting cells with a plurality of agents, each of the plurality of agents not known to have antipsychotic activity;
extracting mRNA from the cells;
determining whether any of the plurality of agents upregulates at least one of the following genes: INSIG1, SCD, FADS2, LDLR, FADS1, FDPS, ACAT2, FDFT1, CYP51A1, FASN, DHCR7, RAB26, TM7SF2, SATB1, FAM117A, GPNMB, NUPR1, VAC14, or LSS, by comparing a level of expression in the extracted mRNA to a level of expression in mRNA extracted from cells not contacted with any of the plurality of agents; and
selecting for use as an antipsychotic any of the plurality of agents determined to upregulate the at least one gene.

2. The method of claim 1 that comprises screening each of the plurality of agents for an ability to upregulate one or more of the following genes: INSIG1, SCD, FADS2, LDLR, FADS1, FDPS, ACAT2, FDFT1, CYP51A1, FASN, DHCR7, RAB26, or TM7SF2.

3. The method of claim 1 that comprises screening each of the plurality of agents for an ability to upregulate one or more of the following genes: INSIG1, SCD, LDLR, FADS1, or CYP51A1.

4. The method of claim 1 that comprises screening each of the plurality of agents for an ability to upregulate one or more of the following genes: LDLR, INSIG1, FADS1, SCD, LSS, CYP51A1, VAC14, NUPR1, GPNMB, FAM117A, or SATB1.

5. The method of claim 1 that comprises screening each of the plurality of agents for an ability to upregulate one or more of the following genes: LDLR, INSIG1, FADS1, SCD, LSS, CYP51A1, or GPNMB.

6. The method of claim 1 that comprises screening each of the plurality of agents for an ability to upregulate each of the following genes: INSIG1, SCD, FADS2, LDLR, FADS1, FDPS, ACAT2, FDFT1, CYP51A1, FASN, DHCR7, RAB26, and TM7SF2.

7. A method for monitoring a patient undergoing treatment with an antipsychotic that comprises monitoring for upregulation of one or more of the following genes in a biological sample of the patient: INSIG1, SCD, FADS2, LDLR, FADS1, FDPS, ACAT2, FDFT1, CYP51A1, FASN, DHCR7, RAB26, TM7SF2, SATB1, FAM117A, GPNMB, NUPR1, VAC14, or LSS.

8. The method of claim 7 that comprises monitoring for upregulation of one or more of the following genes: INSIG1, SCD, FADS2, LDLR, FADS1, FDPS, ACAT2, FDFT1, CYP51A1, FASN, DHCR7, RAB26, or TM7SF2.

9. The method of claim 7 that comprises monitoring for upregulation of one or more of the following genes: INSIG1, SCD, LDLR, FADS1, or CYP51A1.

10. The method of claim 7 that comprises monitoring for upregulation of one or more of the following genes: LDLR, INSIG1, FADS1, SCD, LSS, CYP51A1, VAC14, NUPR1, GPNMB, FAM117A, or SATB1.

11. The method of claim 7 that comprises monitoring for upregulation of one or more of the following genes: LDLR, INSIG1, FADS1, SCD, LSS, CYP51A1, or GPNMB.

12. The method of claim 7 that comprises monitoring for upregulation of each of the following genes: INSIG1, SCD, FADS2, LDLR, FADS1, FDPS, ACAT2, FDFT1, CYP51A1, FASN, DHCR7, RAB26, and TM7SF2.
